# EUROPEAN PATENT APPLICATION

(11) **EP 2 161 579 A1**
(43) Date of publication of application: **10.03.2010**
(21) Application number: 08305501.2
(22) Date of filing: 26.08.2008
(51) Int. Cl.: G01N 33/74, G01N 33/50

(54) **Detection method of biologically active forms of anti-mullerian hormone**

(71) Applicant: INSERM (Institut National de la Santé et de la Recherche Médicale), 75654 Paris Cedex 13 (FR)
(72) Inventor: The designation of the inventor has not yet been filed
(74) Representative: Cabinet Plasseraud

(57) **Abstract**

The present invention relates to a method for detecting or quantifying the presence of at least one biologically active form of Anti-Müllerian Hormone in a sample, and the use of said method for determining the fertility of a subject or for determining and/or monitoring the presence of a cancer in a subject in need thereof and kit for use in said method.

## Description

### FIELD OF THE INVENTION

The invention relates to methods for the detection or quantification of at least one of the biologically active forms of Anti-Müllerian hormone in a sample and their use for determining the fertility of a subject or for diagnosing a cancer in a subject in need thereof.

### BACKGROUND OF THE INVENTION

Anti-Müllerian hormone (AMH), also called Müllerian inhibiting substance (MIS), is a 140 kDa glycoprotein hormone belonging to the transforming growth factor (TGF)-β family of proteins involved in regulating cell growth and differentiation (reviewed in

Chang et al., 2002; Josso et al., 2006). In common with other TGF-β proteins, AMH is synthesized as a large precursor with a short signal sequence followed by the pre- pro hormone that forms homodimers. Prior to secretion, the mature hormone undergoes glycosylation and dimerisation to produce a 140-kDa dimer of identical disulphide-linked 70-kDa monomer subunits; each monomer contains an N-terminal domain (also called the "pro" region) and a C-terminal domain (also called the "mature" region). Less than 10% of AMH is then cleaved at monobasic sites to generate 110-kDa N-terminal and 25-kDa C-terminal homodimers which remain associated in a non-covalent complex. Cleavage can be driven to completion by plasmin in vitro (Pepinsky et al., 1988).

Various studies regarding the biologically active form of AMH led to confusing results: (i) processing was demonstrated to be necessary as mutations at the monobasic sites render AMH resistant to cleavage by plasmin and biologically inactive (Belville et al., 2004), (ii) full-length AMH was as active as cleaved AMH in organ culture assays (di Clemente et al., 1992; Wilson et al., 1993), and (iii) purified C-terminal homodimer was only partially active in organ culture assays, but almost full activity was restored by adding back the N-terminal pro region, which reformed a complex with the mature C-terminus (Wilson et al., 1993).

AMH signals by assembling a transmembrane serine/threonine kinase receptor complex of type I and type II components, resulting in the phosphorylation and activation of latent type I receptor kinase by the constitutively active kinase domain of the type II receptor (Shi and Massagué, 2003). The type II receptor, AMHRII, is AMH specific (Imbeaud et al., 1995; Mishina et al., 1996), while two BMP type I receptors, activin receptor-like kinase (ALK)-2 (Clarke et al., 2001; Visser et al., 2001) and ALK-3 (Jamin et al., 2002), can also serve as redundant type I receptors for AMH (Orvis et al., 2008). When activated by the type II receptor, the type I receptor then phosphorylates the cytoplasmic Smad proteins 1, 5, or 8, which migrate into the nucleus and in concert with other transcription factors, regulate responsive genes, ultimately producing an AMH biological response (reviewed in Josso and di Clemente, 2003).

AMH has an important role in sexual differentiation during development. AMH is produced by the Sertoli cells of the testis in the male, and by ovarian granulosa cells in the female. During fetal development in males, secretion of AMH from testicular Sertoli cells is essential for the regression of the Mullerian ducts, and thus the normal male reproductive tract development (Blanchard and Josso, 1974; Josso et al., 2006). In the male, secretion of AMH by the Sertoli cells starts during embryogenesis and continues throughout life. Levels drop following puberty, decreasing slowly to a relatively low post-puberty value.

The Mullerian ducts are the primordium for the uterus, Fallopian tubes, and upper part of the vagina in the female (Behringer et al., 1994). In the female, serum AMH is maintained at relatively low levels when compared to the male. After puberty, when menstrual cycling begins, circulating AMH slowly decreases throughout life and becomes undetectable at menopause.

AMH is associated with female reproductive biology. AMH is known to be a measure of ovarian reserve and has been suggested as a means to control female fertility. AMH has considerably gained interest in the recent time in the field of assisted reproduction as a predictor of ovarian response and pregnancy. Serum AMH levels seems to be more strongly related to ovarian follicular status and to be a more discriminatory marker of assisted reproductive technology (ART) outcome than follicle stimulating hormone (FSH), inhibin B and oestradiol, which are more commonly used markers. Assessment of ovarian reserve and ovarian responsiveness in women undergoing ART is useful in optimising the treatment protocol. Availability of a reliable measure of ovarian reserve and ovarian responsiveness which are indicators of female fertility is therefore essential.

AMH is also associated with male reproductive biology. Indeed, it has been suggested that levels of seminal and/or serum AMH are decreased in men with male factor infertility but conflicting data have been reported (Tuttelman et al., 2008). Measurement of the bioactive fraction of AMH in biological fluids could perhaps resolve the contreversy. Thus, availability of a reliable measure of AMH which is an indicator of male fertility is also of great interest.

Ovarian cancer comprises a heterogeneous group of tumours of three major types: epithelial, germ cell and sex cord stromal. Granulosa cell tumours (GCT) represent 3% of all ovarian tumours, but account for 6-10% of malignant tumours of the ovary. Although the malignant potential of GCT is relatively low, these tumours frequently recur and spread in the pelvis. Late recurrences, appearing up to 20 years after the initial treatment, are frequent and have a poor prognosis, below than 35%.

The initial treatment of GCT is mainly surgical. In advanced stages or in recurrences, when the tumour is not confined to the ovaries, complete surgical removal may be difficult, and adjuvant chemotherapy and/or radiotherapy can be used. However, as GCT have low radio- and chemosensitivity, the success of the treatment is dependent on an early diagnosis of the recurrence, when complete removal of the tumour is still possible.

GCT are hormone-secreting neoplasms; they may produce steroids, such as oestradiol and progesterone, and they secrete peptide hormones, such as inhibin and AMH. Because serum levels of AMH and inhibin are increased in patients with GCT of the ovary, both hormones are used as biochemical markers of the disease (Long et al., 2000; Gustafson et al., 1992).

AMH has also been proposed as a marker of breast cancer, uterine cancer and prostate cancer (Hoshiya et al., 2003; Renaud et al. PNAS 2005).

Methods for measuring AMH in a sample are thus of great interest for diagnosis and/or monitoring a cancer or for determining the fertility of a subject.

Existing methods for measuring AMH in a sample are described in WO2006/127850.

One of these methods, named the Hudson assay, uses two monoclonal antibodies raised against human recombinant AMH, both being directed to epitopes in the pro region of the molecule.

Another assay employed in AMH detection uses a pair of monoclonal antibodies, one of which being directed to an epitope in the pro region and the other one being directed in the mature region of AMH.

The patent application WO2006/127850 describes another assay for measuring AMH in a sample, based on the use of two antibodies, one directed to a first epitope in the mature region and the other one directed to a second epitope in the mature region.

There is still a need to provide a more accurate assay for measuring AMH in a sample.

### SUMMARY OF THE INVENTION

One object of the invention is a method for detecting or quantifying the presence of at least one biologically active form of Anti-Müllerian Hormone (AMH) in a sample, said method comprising detecting the presence of cleaved AMH or C-terminal AMH in said sample.

Another object of this invention is a method for determining the fertility of a subject or for determining and/or monitoring the presence of a cancer in a subject in need thereof, said method comprising quantifying at least one biologically active form of AMH in a sample obtained from said subject.

Another object of the invention is a kit for use in said method, comprising as separate components:
- a binding partner comprising the extra-cellular domain of AMHRII, and
- an anti-AMH antibody that does not prevent the interaction between the biologically active forms of AMH and the extra-cellular domain of AMHRII.

Another object of this invention is the use of at least one biologically active form of AMH for treating cancer.

### DETAILED DESCRIPTION OF THE INVENTION

While working on how AMH interacts with its type I and type II receptors, the inventors made the following observations:
(i) AMH should be cleaved for it to bind AMH receptor II (AMHRII),
(ii) dissociation of the pro region from the mature C-terminus is not required for AMH interaction for AMHRII,
(iii) cleaved non-covalent complex and the C-terminal homodimer bind to AMHRII and stimulate phosphorylation of Smads and
(iv) the C-terminal AMH is more potent than cleaved AMH in stimulating Smad phosphorylation.

In summary, the inventors demonstrated that the biological active forms of AMH are cleaved AMH and C-terminal AMH.

These observations led the inventors to provide an improved method for measuring AMH in a sample, one particular advantage of this method being that it allows the measure of the biologically active forms of AMH (cleaved AMH and C-terminal AMH) in a sample. Such a method is therefore of great interest as measuring the presence of the biologically active forms of AMH is more relevant than measuring all forms of AMH for the diagnosis applications.

The invention thus relates to a method for detecting or quantifying the presence of at least one biologically active form of Anti- Müllerian Hormone (AMH) in a sample, said method comprising detecting the presence of cleaved AMH or C-terminal AMH in said sample.

The invention also relates to a method for determining the fertility of a subject or for determining and/or monitoring a cancer in a subject, said method comprising measuring the biologically active forms of AMH in a sample.

### Definitions

As used herein, the term "Anti-Müllerian Hormone" (AMH) corresponds to a 140 kDa glycoprotein hormone. AMH is synthesized as a large precursor with a short signal sequence followed by the pre-pro hormone that forms homodimers. Prior to secretion, the mature hormone undergoes glycosylation and dimerisation to produce a 140-kDa dimer of identical disulphide-linked 70-kDa monomer subunits; each monomer contains an N-terminal domain (also called the "pro" region) and a C-terminal domain (also called the "mature" region).

"Full-length AMH" as used herein corresponds to the 140-kDa dimer of identical disulphide-linked 70-kDa monomer subunits; each monomer contains an N-terminal domain (also called the "pro" region) and a C-terminal domain (also called the "mature" region).

Less than 10% of AMH is then cleaved at monobasic sites to generate 110-kDa N-terminal and 25-kDa C-terminal homodimers which remain associated in a non-covalent complex. Thus full length AMH produced by cells contains about 90% 140 kDa homodimer and about 10% cleaved non-covalent complex.

"Cleaved AMH" as used herein corresponds to the 110-kDa N-terminal and 25-kDa C-terminal homodimers which remain associated in a non-covalent complex, as defined in Pepinsky et al., 1988.

"N-terminal AMH" as used herein corresponds to the 110-kDa N-terminal homodimer, as defined in Pepinsky et al., 1988.

"C-terminal AMH" as used herein corresponds to the 25-kDa C-terminal homodimer, as defined in Pepinsky et al., 1988.

As used herein, the term "subject" denotes a mammal, such as a rodent, a feline, a canine, and a primate. Preferably a subject according to the invention is a human.

As used herein, the term "antibody" refers to a protein capable of specifically binding an antigen, typically and preferably by binding an epitope or antigenic determinant or said antigen. The term "antibody" also includes recombinant proteins comprising the binding domains, as well as variants and fragments of antibodies. Examples of fragments of antibodies include Fv, Fab, Fab', F(ab')2, dsFv, scFv, sc(Fv)2, diabodies and multispecific antibodies formed from antibody fragments.

"Function-conservative variants" as used herein refer to those in which a given amino acid residue in a protein or enzyme has been changed (inserted, deleted or substituted) without altering the overall conformation and function of the polypeptide. Such variants include protein having amino acid alterations such as deletions, insertions and/or substitutions. A "deletion" refers to the absence of one or more amino acids in the protein. An "insertion" refers to the addition of one or more of amino acids in the protein. A "substitution" refers to the replacement of one or more amino acids by another amino acid residue in the protein. Typically, a given amino acid is replaced by an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared. Two amino acid sequences are "substantially homologous" or "substantially similar" when greater than 80 %, preferably greater than 85 %, preferably greater than 90 % of the amino acids are identical, or greater than about 90 %, preferably greater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

As used herein, "measuring" encompasses detecting or quantifying. As used herein, "detecting" means determining if at least one biologically active form of AMH is present or not in a sample. As used herein, "quantifying" means determining the amount of at least one biologically active form of AMH in a sample.

### The invention

One object of the invention is a method for detecting or quantifying the presence of at least one biologically active form of Anti-Müllerian Hormone (AMH) in a sample, said method comprising detecting the presence of cleaved AMH or C-terminal AMH in said sample.

Thus, said method comprises the detection of only cleaved AMH and/or C-terminal AMH in the sample, and detection of cleaved AMH and/or C-terminal AMH does not allow the detection of full-length AMH and N-terminal AMH.

In one embodiment of the invention, said method comprises detecting at least one biologically active form of AMH in a sample obtained from said subject with a binding partner comprising the extra-cellular domain of type II AMH receptor (AMHRII).

According to the invention, the extra-cellular domain of AMHRII corresponds to residues 18-145 of Swiss Prot Accession Q16671 and functional-conservative variants or fragments thereof that retain the function of the extra-cellular domain of AMHRII, which is the capacity of binding the biologically active forms of AMH: cleaved AMH or C-terminal AMH.

The capacity of a variant or fragment of the extra-cellular domain of AMHRII to bind cleaved AMH or C-terminal AMH may be assessed by any conventional techniques known in the art. Examples of said conventional techniques are precipitation experiments and

ELISA experiments as described here after in the experimental procedures.

According to another embodiment of the invention, the binding partner may be a fusion protein comprising the extra-cellular domain of type II AMH receptor (AMHRII), fused directly or indirectly to another protein. Techniques for producing and purifying said fusion protein are well known in the art. For example, when the fusion is indirect, a peptide linker may be used such as IEGRMD.

Typically, an example of such fusion protein is an AMHRII-Fc fusion protein comprising the extra-cellular domain of AMHRII as defined here above, fused to at least a domain of the Fc region of an immunoglobulin. In this AMRHII-Fc fusion protein, the extra-cellular domain of AMHRII forms the amino-terminal domain of the fusion protein and the Fc region forms the carboxy-terminal domain of the fusion protein. The extra-cellular domain of AMHRII may be fused directly or indirectly to the Fc region.

In one embodiment, said fusion protein comprises the mature extra-cellular domain of AMHRII as defined here above, fused to the CH2 and CH3 domains and the hinge region of IgG1 (residues 104-330 of Swiss Prot Accession P01857).

According to the invention, the sample susceptible to contain biological active forms of AMH is a biological sample, such as cell lysates or culture medium, or is a body fluid such as whole blood, serum, plasma, follicular fluid, seminal fluid, synovial fluid, cerebrospinal fluid, saliva, or urine.

In a preferred embodiment, said sample is a serum sample, a whole blood sample, a plasma sample, a follicular fluid sample, a seminal fluid sample.

According to the invention, the detection or quantification of at least one biologically active form of AMH is achieved by any methods known in the art using a binding partner comprising the extra-cellular domain of AMHRII. Examples of said methods include, but are not limited to, standard electrophoretic and immunodiagnostic techniques such as western blots, radioimmunoassay, ELISA (enzyme-linked immunosorbant assay), "sandwich" immunoassay, immunoradiometric assay, gel diffusion precipitation reaction, immunodiffusion assay, precipitation reaction, agglutination assay (such as gel agglutination assay, hemagglutination assay, etc.), complement fixation assay, immunofluorescence assay, protein A assay, immunoelectrophoresis assay, high performance liquid chromatography, size exclusion chromatography, solid-phase affinity, etc.

According to the invention, the binding partner comprising the extra-cellular domain of AMHRII may be labelled with a detectable molecule or substance. Examples of suitable labels for this purpose include a chemiluminescent agent, a colorimetric agent, an energy transfer agent, an enzyme, a substrate of an enzymatic reaction, a fluorescent agent, or a radioisotope. The label may be coupled directly or indirectly by any known method in the art.

In one embodiment, the detection or quantification of at least one of the biologically active forms of AMH in a sample may be achieved by a protein chip array system, wherein the binding partner comprising the extra-cellular domain of AMHRII is coated directly or indirectly on a protein chip array. The sample to be tested is labelled by biotinylation in vitro. Biotinylated cleaved AMH and C-terminal AMH trapped on the array are then detected by avidin or streptavidin which strongly binds biotin. If avidin is conjugated with horseradish peroxidase or alkaline phosphatase, the captured cleaved AMH and C-terminal AMH can be visualized by enhanced chemical luminescence. The amount of protein bound to the extra-cellular domain of AMHRII represents the level of cleaved AMH and C-terminal AMH in the sample. Other methods, like immunochemical staining, surface plasmon resonance, matrix-assisted laser desorption/ionization-time of flight, can also be used to detect the captured proteins.

In another embodiment of the invention, the detection or quantification of at least one of the biologically active forms of AMH in a sample may be achieved by a cytometric bead array system wherein the binding partner comprising the extra-cellular domain of AMHRII is coated directly or indirectly on beads.

In another embodiment of the invention, the detection or quantification of at least one of the biologically active forms of AMH in a sample may be achieved by a competitive immunoassay. Example of competitive immunoassays include enzyme immunoassay or enzyme-linked immunoassay (EIA or ELISA), fluorescent immunoassay, radiometric or radioimmunoassay (RIA), magnetic separation assay (MSA), lateral flow assay, diffusion immunoassay, immunoprecipitation immunoassay, immunosorbent or "antigen-down" assay using at least one of the biologically active form of AMH bound to a solid support, or agglutination assay.

In one example of competitive immunoassay, the quantification of at least one of the biologically active forms of AMH is achieved by
- combining a sample containing AMH with a known amount of a labelled the biologically active form of AMH to create a spiked sample,
- binding labelled and unlabelled biologically active form of AMH in the spiked sample with a binding partner comprising the extra-cellular domain of AMHRII, wherein the binding partner binds to the biologically active forms of AMH to create complexes and to labelled biologically active forms of AMH to create labelled complexes,
- measuring the amount of labelled complex
- calculating the amount of the biologically active forms of AMH present in the sample.

In one embodiment, said binding partner comprising the extra-cellular domain of AMHRII may be coated to a solid support, said solid support comprising a protein binding surface such as a microtiter plate, a colloid metal particle, an iron oxide particle, a latex particle or a polymeric bead.

In one embodiment, the labelled biologically active forms of AMH may comprise a label such as a chemiluminescent agent, a colorimetric agent, an energy transfer agent, an enzyme, a fluorescent agent, or a radioisotope. Examples of chemiluminescent agent include an enzyme that produces a chemiluminescent signal in the presence of a substrate(s) that produce chemiluminescent energy when reacted with the enzyme.

Examples of such an enzyme include horseradish peroxidase (HRP) and alkaline phosphatise (AP). Other examples of a chemiluminescent agent include a non-enzymatic direct chemiluminescent label, such as Acrinidium ester system. Examples of a colorimetric agent include an enzyme such as horseradish peroxidase, alkaline phosphatase, and acetylcholine esterase (AChE). Examples of energy transfer agent include fluorescent lanthanide chelates. Examples of fluorescent agents include fluorescent dyes. Examples of radioisotopes include ¹²⁵I, ¹⁴C and ³H.

While the spiked sample is incubated with the binding partner, the biologically active forms of AMH present in the sample and the added labelled biologically active forms of AMH compete for binding to the available extra-cellular domain of AMHRII. Labelled biologically active forms of AMH will then be able to bind the binding partner depending on the relative concentration of the unlabeled biologically active forms of AMH present in the sample. Thus, when the amount of labelled biologically active forms of AMH is measured, it is inversely proportional to the amount of unlabeled biologically active forms of AMH present in the sample. The amount of biologically active forms of AMH present in the sample may then be calculated based on the amount of labelled biologically active forms of AMH measured, using standard techniques.

In another example of competitive immunoassay, the quantification of at least one of the biologically active forms of AMH is achieved by the binding partner coupled with or conjugated with a ligand, said ligand binding to an additional antibody added to the sample. One example of said ligand is fluorescein. The additional antibody may be bound to a solid support. In this example of competitive immunoassay, the additional antibody binds to the ligand coupled with the binding partner that binds in turn to (i) the biologically active forms of AMH present in the sample and (ii) labelled the biologically active forms of AMH added to the sample. Said mass complex formed allows isolation and measurement of the signal generated by the label coupled with the labelled the biologically active forms of AMH.

In another example of competitive immunoassay, the biologically active forms of AMH may be bound to a solid support, and incubated with (i) the binding partner and (ii) a sample containing the biologically active forms of AMH to be measured. The binding partner binds either the biologically active forms of AMH bound to the solid support or the biologically active forms of AMH present in the sample, in relative proportion depending of the concentration of the biologically active forms of AMH present in the sample. The binding partner that binds to the biologically active forms of AMH bound to the solid support is then bound to another antibody that is coupled with a label. While the binding partner is the extra-cellular domain of AMHRII, the other antibody coupled with a label may be an antibody directed to the extra-cellular domain of AMHRII, which does not prevent the interaction of the biologically active forms of AMH with the extra-cellular domain of AMHRII. While the binding partner is a fusion protein comprising the extra-cellular domain of AMHRII, the other antibody may be an antibody directed to the protein fused to the extra-cellular domain of AMHRII (for example an antibody anti-Fc while the fusion protein is an AMHRII-Fc fusion protein). The amount of signal generated from the label is then detected to measure the amount of extra-cellular domain of AMHRII bound. Such a measurement will be inversely proportional to the amount of the biologically active forms of AMH present in the sample. Such an assay may be used in a microtiter plate.

In another example of competitive immunoassay, the biologically active forms of AMH to be measured compete with the biologically active forms of AMH that is bound to a first solid support particle, such as Ficoll, for the binding partner that is bound to or coated to a second solid support particle. Cross-binding or agglutination between the particles occurs and forms clumps of co-agglutination lattice. Alternatively, the binding partner binds to the free biologically active forms of AMH in the sample, so that the amount of agglutination is inversely proportional to the amount of biologically active forms of AMH in the sample. The amount of agglutination may be measured using standard techniques, such as spectrophotometry.

In another embodiment of the invention, the quantification of at least one of the biologically active forms of AMH in a sample may be achieved by a non-competitive immunoassay referred as immunometric, "two-site" or "sandwich" immunoassays, wherein the biologically active forms of AMH may be bound to or sandwiched between an anti-AMH antibody and a binding partner comprising the extra-cellular domain of AMHRII.

Examples of competitive immunoassays include enzyme immunoassay or enzyme-linked immunoassay (EIA or ELISA), fluorescent immunoassay, radiometric or radioimmunoassay (RIA), magnetic separation assay (MSA), lateral flow assay, diffusion immunoassay, immunoprecipitation immunoassay, immunosorbent or "antigen-down" assay using cleaved AMH and C-terminal AMH bound to a solid support, or agglutination assay.

In this embodiment, the quantification of at least one of biologically active forms of AMH in a sample is achieved by
- contacting said sample with
   ○ a binding partner comprising the extra-cellular domain of AMHRII, and
   ○ an anti-AMH antibody that does not prevent the interaction between the biologically active forms of AMH and the binding partner,
- measuring the amount of bound anti-AMH antibody or bound binding partner and
- calculating the amount of at least one of biologically active forms of AMH in the sample.

In one embodiment, a one-step assay (simultaneous incubation of the binding partner and the anti-AMH antibody) is useful. In another embodiment, a two-step assay (sequential incubation of the binding partner and the anti-AMH antibody) is useful. A two-assay is preferred in the case where other molecules could compete for binding to the binding partner.

In one embodiment, while the anti-AMH antibody is the "capture" antibody, it is bound to a solid support, such as protein coupling or protein binding surface, colloid metal particles, iron oxide particles, or polymeric beads. One example of polymeric beads is a latex particle. In such an embodiment, the capture antibody is bound to or coated on a solid phase support using standard non-covalent or covalent binding methods, depending on the required analytical and/or solid phase separation requirements. The solid support may be in the form of test-tubes, beads, microparticles, filter paper, membrane, glass filters, magnetic particles, glass or silicon chips or other materials known in the art. The use of microparticles, particularly magnetisable particles, that have been directly coated with the antibody or particles that have been labelled with a universal binder (such as avidin or anti-species antibody) is useful for significantly shortening the assay incubation time.

Alternatively, the capture anti-AMH antibody may be coupled with a ligand that is recognised by an additional antibody that is bound to or coated on the solid support. Binding of the capture antibody to the additional antibody via the ligand then indirectly immobilizes the capture antibody on the support. An example of such a ligand is fluorescein.

In said embodiment, the binding partner comprising the extra-cellular domain of AMHRII is thus coupled or conjugated with a label using procedures known in the art. Examples of suitable labels for this purpose include a chemiluminescent agent, a colorimetric agent, an energy transfer agent, an enzyme, a substrate of an enzymatic reaction, a fluorescent agent, or a radioisotope.

Alternatively, the binding partner may also be detected indirectly by a secondary detection system. Said secondary detection system is based on several different principles known in the art such as antibody recognition and other forms of immunological or non-immunological bridging and signal amplification detection systems (for example, the biotin-streptavidin system).

In one embodiment, while the binding partner is the extra-cellular domain of AMHRII, a labelled antibody directed to the extra-cellular domain of AMHRII may be used as secondary detection system, said antibody being still capable of binding to the extra-cellular domain of AMHRII, when the biologically active forms of AMH and the extra-cellular domain of AMHRII are already interacting.

The capacity of an antibody directed to the extra-cellular domain of AMHRII to bind the extra-cellular domain of AMHRII, when the biologically active forms of AMH and the extra-cellular domain of AMHRII are already interacting, may be assessed by any conventional techniques known in the art. Examples of said conventional techniques are ELISA experiments as described here after in the experimental procedures. For example, an anti-AMH antibody directed to an epitope in the N-terminal region of AMH may be coated to a solid support and then contacted with biologically active forms of AMH to form complexes. Said complexes are then contacted with the extra-cellular domain of AMHRII, to create "sandwich" complexes. Said "sandwich" complexes are finally contacted with the labelled antibody directed to the extra-cellular domain of AMHRII. After washing, detection of the label is indicative of the capacity of the antibody directed to the extra-cellular domain of AMHRII to bind to the extra-cellular domain of AMHRII, while the extra-cellular domain of AMHRII is already complexed with a biologically active form of AMH.

In another embodiment, while the binding partner is a fusion protein, a labelled antibody directed to the protein fused to the extra-cellular domain of AMHRII may be used as secondary detection system. For example, while the binding partner is an AMHRII-Fc fusion protein, a labelled antibody anti-Fc may be used as secondary detection system.

In another embodiment, when a signal amplification system is used, the label includes a first protein such as biotin coupled with the binding partner, and a second protein such as streptavidin that is coupled with an enzyme. The second protein binds to the first protein. The enzyme produces a detectable signal when provided with substrate(s), so that the amount of signal measured corresponds to the amount of binding partner that is bound to biologically active forms of AMH. Examples of enzymes include, without limitation, alkaline phosphatase, amylase, luciferase, catalase, beta-galactosidase, glucose oxidase, glucose-6-phosphate dehydrogenase, hexokinase, horseradish peroxidase, lactamase, urease and malate dehydrogenase. Suitable substrates include, without limitation, TMB (3,3',5,5'-tetramethyl benzidine), OPD (o-phenylene diamine), and ABTS (2,2'-azino-bis (3-ethylbenzthiozoline-6-sulfonic acid). The signal amplification approach may be used to significantly increase the assay sensitivity and low level reproducibility and performance.

In an alternative embodiment, the anti-AMH antibody may be labelled and used as the "detection" antibody and the binding partner is used as the "capture" antibody.

In one embodiment of this alternative embodiment where the binding partner comprising the extra-cellular domain of AMHRII is bound to a solid support, another binding partner comprising the extra-cellular domain of AMHRI (AMH type I receptor) may also be bound to said solid support.

In said embodiment, the extra-cellular domain of activin receptor-like kinase (ALK)-2 (Clarke et al. 2001; Visser et al. 2001) or ALK-3 (Jamin et al. 2002) may be used as extra-cellular domain of AMHRI (Orvis et al. 2008).

In a preferred embodiment, the extra-cellular domain of ALK-3 is used as the extra-cellular domain of AMHRI.

According to the invention, the extra-cellular domain of AMHRI corresponds to residues 24-152 of Swiss Prot Accession P36894 for ALK-3 and 21-123 of Swiss Prot Accession Q04771 for ALK-2 and functional-conservative variants or fragments thereof that retain the function of the extra-cellular domain of AMHRI, which is the capacity of binding the biologically active forms of AMH: cleaved AMH or C-terminal AMH.

According to another embodiment of the invention, the binding partner may be a fusion protein comprising the extra-cellular domain of AMHRI, fused directly or indirectly to another protein, as described here above for the binding partner comprising the extra-cellular domain of AMHRII.

In one embodiment, the anti-AMH antibody is an antibody directed to an epitope directed to the N-terminal region of AMH. While the antibody used is directed to the N-terminal region of AMH, the biologically active form of AMH detected and measured is only the cleaved AMH, as the C-terminal AMH can not be detected by such an antibody.

Typically, an example of anti-AMH antibody directed to an epitope directed to the N-terminal region of AMH is the mouse anti-AMH monoclonal mAb 10.6.

In another embodiment, the anti-AMH antibody is an antibody directed to an epitope directed to the C-terminal region of AMH, which does not prevent the interaction between biologically active forms of AMH and the extra-cellular domain of AMHRII.

Typically, an example of anti-AMH antibody directed to an epitope directed to the C-terminal region of AMH, which does not prevent the interaction between biologically active forms of AMH and the extra-cellular domain of AMHRII is the mouse monoclonal mAb 22A2.

In another embodiment, the anti-AMH antibody comprises a mixture of both antibodies as described here above.

Antibodies useful in the various embodiments of the invention encompass commercially available antibodies and antibody fragments, as well as any novel antibodies generated to bind to a suitable epitope on AMH. The antibodies used in various embodiments exemplified herein are monoclonal or polyclonal in nature. Other antibodies and antibody fragments, such as recombinant antibodies, chimeric antibodies, humanised antibodies, Fab or Fv fragments are also useful.

The capacity of an antibody directed to the C-terminal domain of AMH to not prevent the interaction between the extra-cellular domain of AMHRII and the biologically active forms of AMH, may be assessed by any conventional techniques known in the art. Examples of said conventional techniques are ELISA experiments as described here after in the experimental procedures. For example, said anti-AMH antibody directed to an epitope in the C-terminal region of AMH may be coated to a solid support and then contacted with biologically active forms of AMH to form complexes. Said complexes are then contacted with labelled extra-cellular domain of AMHRII, to create "sandwich" complexes. After washing, detection of the label is indicative of the capacity of the antibody directed to an epitope in the C-terminal region of AMH to not prevent the interaction between the extra-cellular domain of AMHRII and the biologically active forms of AMH.

Another object of the invention is a method for determining the fertility of a subject or for diagnosing and/or monitoring the presence of a cancer in a subject in need thereof, said method comprising quantifying at least one biologically active form of Anti-Müllerian Hormone (AMH) in a sample obtained from said subject, as described here above.

Typically, said cancer is an AMH type II receptor-expressing cancer.

In one embodiment, said method is intended for diagnosing and/or monitoring a cancer, said cancer being a neoplasm stemming from granulosa cell tumours, an ovarian cancer, a breast cancer, a uterine cancer or a prostate cancer.

In a preferred embodiment, said cancer is an ovarian cancer steaming from granulosa cell tumours. As inhibin has also been reported as a marker of an ovarian cancer steaming from granulosa cell tumours, the quantification of the biological forms of AMH may also be coupled to the quantification of inhibin to diagnose and/or monitor an ovarian cancer.

In another embodiment, said method is intended for determining the fertility of a subject.

In one embodiment, the method of the invention is intended for determining the fertility of female subject.

Inhibin, estradiol and follicle stimulating hormone (FSH) have also been described as potential markers of the fertility of a female subject. Thus, the quantification of the biological forms of AMH may also be coupled to the quantification of inhibin, oestradiol and FSH for determining the fertility of a female subject.

In another embodiment, the method of the invention is intended for determining the fertility of a male subject.

Another object of the invention is a method for diagnosing persistent müllerian duct syndrome (PMDS) in a subject in need thereof, said method comprising quantifying at least one biologically active form of Anti-Müllerian Hormone (AMH) in a sample obtained from said subject, as described here above.

According to the invention, said method is for diagnosing PMDS that are caused by mutation(s) in AMH gene.

According to the invention, said method for determining the fertility of a subject or for diagnosing and/or monitoring the presence of a cancer or for diagnosing PMDS in a subject in need thereof, comprises the steps of:
- providing a sample obtained from a subject,
- contacting said sample with a binding partner comprising the extra-cellular domain of AMHRII as defined here above under conditions appropriate for formation of a complex between said binding partner and the biologically active forms of AMH present in the sample,
- quantifying the amount of complexes formed to determine the amount of biologically active forms of AMH present in the sample,
- correlating said amount of biologically active forms of AMH with the determination of the fertility of a subject or with the diagnosis and/or the monitoring of a cancer or with the diagnosis of PMDS in a subject.

The amount of biologically active forms of AMH quantified may thus be compared with the corresponding amount detected in the samples of control subjects, in previous samples obtained from the subject or with normal reference values.

While the method of the invention is intended for the diagnosis of a cancer, control subjects are for example subjects that have not been diagnosed for said cancer.

Normal reference values refer to the amount of at least one of the biological forms of AMH that can be determined by the method of the invention in a subject that has not been diagnosed for a cancer or in a subject that is considered as being fertile or in a subject that has not been diagnosed for PMDS.

A high amount of biologically active forms of AMH in a sample compared to the control amount is indicative of a fertility of a subject, whereas a lower amount is a marker of infertility.

A high amount of biologically active forms of AMH in a sample compared to the control amount is indicative of the presence of a cancer.

Quantifying the amount of biologically active forms of AMH is also of interest for monitoring for example the efficacy of surgery and cancer recurrence. Hence, serum AMH levels usually normalizes within few days following surgery, thus persistent AMH levels are indicative of residual cancer.

A low amount of biologically active forms of AMH in a sample compared to the control amount is indicative of the presence of PMDS.

Another object of the invention is a kit for use in the method of the invention as described here above, said kit comprising, as separate components,
- a binding partner comprising the extra-cellular domain of AMHRII, and
- an anti-AMH antibody that does not prevent the interaction between the biologically active forms of AMH and the extra-cellular domain of AMHRII.

In one embodiment, said binding partner is the extra-cellular domain of AMHRII as defined here above.

In another embodiment, said binding partner is a fusion protein comprising the extra-cellular domain of AMHRII fused to another protein.

In another embodiment, said binding partner is an AMHRII-Fc fusion protein as described here above.

In one embodiment, said binding partner may be coated to a solid support. Suitable examples of solid support are identified here above.

In another embodiment, said binding partner may be labelled. Suitable examples of labels are similarly identified here above.

In one embodiment, said anti-AMH antibody is directed to an epitope in the N-terminal domain of AMH.

In another embodiment, said anti-AMH antibody is directed to an epitope in the C-terminal domain of AMH.

The capacity of an antibody directed to the C-terminal domain of AMH to not prevent the interaction between the extra-cellular domain of AMHRII and the biologically active forms of AMH, may be assessed by any conventional techniques known in the art. Examples of said conventional techniques are ELISA experiments as described here after in the experimental procedures. For example, said anti-AMH antibody directed to an epitope in the C-terminal region of AMH may be coated to a solid support and then contacted with biologically active forms of AMH to form complexes. Said complexes are then contacted with labelled extra-cellular domain of AMHRII, to create "sandwich" complexes. After washing, detection of the label is indicative of the capacity of the antibody directed to an epitope in the C-terminal region of AMH to not prevent the interaction between the extra-cellular domain of AMHRII and the biologically active forms of AMH.

In another embodiment, said anti-AMH antibody may be coated to a solid support as described here above.

In another embodiment, said anti-AMH antibody may be labelled as described here above.

The kit may also contain optional additional components for performing the method of the invention. Such optional components are for example containers, mixers, buffers, instructions for assay performance, labels, supports, and reagents necessary to couple the antibody or the binding partner to the support or label.

Various studies have suggested the use of AMH as an anti-cancer agent. In particular, they suggested the use of AMH as a therapeutic agent for treating ovarian cancer as AMH was described as a powerful inhibitor of granulosa cell proliferation (Pieretti-Vanmarcke et al., 2006a; Pieretti-Vanmarcke et al., 2006b; La Marca et al., 2007).

Another object of the invention is the use of at least one biologically active form of AMH for treating cancer in a subject in need thereof.

Another object of the invention is the use of at least one biologically active form of AMH as an anti-cancer drug.

Another object of the invention is cleaved AMH and/or C-terminal AMH for treating cancer in a subject in need thereof.

Another object of the invention is cleaved AMH and/or C-terminal AMH for use as an anti-cancer drug for treating cancer in a subject in need thereof.

Another object of the invention is the use of cleaved AMH and/or C-terminal AMH for the manufacture of a medicament for treating cancer in a subject in need thereof.

In one embodiment, the use of at least one biologically active form of AMH is intended for treating an AMH type II receptor-expressing cancer.

In another embodiment, the use of at least one biologically active form of AMH is intended for treating a cancer among a gynaecological cancer, a neural crest cancer, a breast cancer, or a prostate cancer.

In another embodiment, the use of at least one biologically active form of AMH is intended for treating a cancer among a neoplasm stemming from granulosa cell tumours, an ovarian cancer, or a uterine cancer.

In another embodiment, the use of at least one biologically active form of AMH is intended for treating a melanoma.

In a preferred embodiment, said cancer is an ovarian cancer steaming from granulosa cell tumours.

In another embodiment, at least one biologically active form of AMH is used as an anti-cancer agent in a combined therapy with a least one chemotherapeutic agent such as paclitaxel, cisplatin, doxorubicin, AzadC or rapamycin.

Persistent müllerian duct syndrome (PMDS) refers to the presence of a uterus and sometimes other müllerian duct derivatives in a male. Typical features include undescended testes (cryptorchidism) and the presence of a small, underdeveloped uterus in a male infant or adult. Symptoms of PMDS include a bulge in the inguinal canal of a male infant due to herniation of the uterus. The presence of a uterus in a male subject can be detected by ultrasound or MRI of the pelvis. This condition is usually caused by deficiency of fetal AMH effect due to mutations of the gene for AMH or its receptor.

Another object of the invention is cleaved AMH and/or C-terminal AMH for treating persistent müllerian duct syndrome (PMDS) in a subject in need thereof.

Another object of the invention is cleaved AMH and/or C-terminal AMH for use as an anti-cancer drug for treating PMDS in a subject in need thereof.

Another object of the invention is the use of cleaved AMH and/or C-terminal AMH for the manufacture of a medicament for treating PMDS in a subject in need thereof.

In addition to administration of cleaved AMH and/or C-terminal AMH as described here above, surgery (orchiopexy) can be performed to retrieve the testes and position them in the scrotum is the primary treatment. During this surgery, the uterus is usually removed and attempts made to dissect away müllerian tissue from the vas deferens and epididymis for the purpose of improving the chance of fertility. In the event that the testes are damaged or lost, testosterone replacement therapy can be administered at puberty.

Endometriosis is a common condition of women in which the tissue that lines the uterus is found to be growing outside the uterus, on or in other areas of the body. The endometriotic tissues still detach and bleed, but the result is far different: internal bleeding, degenerated blood and tissue shedding, inflammation of the surrounding areas, pain, and formation of scar tissue may result. In addition, depending on the location of the growths, interference with the normal function of the bowel, bladder, small intestines and other organs within the pelvic cavity can occur. In very rare cases, endometriosis has also been found in the skin, the lungs, the diaphragm, and even the brain.

Therefore, as AMH has been described to have the ability to inhibit growth of tissue derived from the Müllerian ducts, AMH may be used in the treatment of endometriosis.

Another object of the invention is cleaved AMH and/or C-terminal AMH for treating endometriosis in a subject in need thereof.

Another object of the invention is cleaved AMH and/or C-terminal AMH for use as an anti-cancer drug for treating endometriosis in a subject in need thereof.

Another object of the invention is the use of cleaved AMH and/or C-terminal AMH for the manufacture of a medicament for treating endometriosis in a subject in need thereof.

Another object of the invention is a method for treating cancer in a subject in need thereof, said method comprising administering a therapeutically effective amount of cleaved AMH and/or C-terminal AMH to said subject.

In one embodiment, said method is intended for treating an AMH type II receptor-expressing cancer.

In another embodiment, said method is intended for treating a cancer among a gynaecological cancer, a neural crest cancer, a breast cancer, or a prostate cancer.

In another embodiment, said method is intended for treating a cancer among a neoplasm stemming from granulosa cell tumours, an ovarian cancer, or a uterine cancer.

In another embodiment, said method is intended for treating a melanoma.

In a preferred embodiment, said cancer is an ovarian cancer steaming from granulosa cell tumours.

In another embodiment, said method of administering a therapeutically effective amount of cleaved AMH and/or C-terminal AMH to the subject is used in a combined therapy with a least one chemotherapeutic agent such as paclitaxel, cisplatin, doxorubicin, AzadC or rapamycin.

Another object of the invention is a method for treating persistent müllerian duct syndrome (PMDS) in a subject in need thereof, said method comprising administering a therapeutically effective amount of cleaved AMH and/or C-terminal AMH to said subject.

Another object of the invention is a method for treating endometriosis in a subject in need thereof, said method comprising administering a therapeutically effective amount of cleaved AMH and/or C-terminal AMH to said subject.

The following examples are given for the purpose of illustrating various embodiments of the invention.

### BRIEF DESCRIPTION OF THE FIGURES

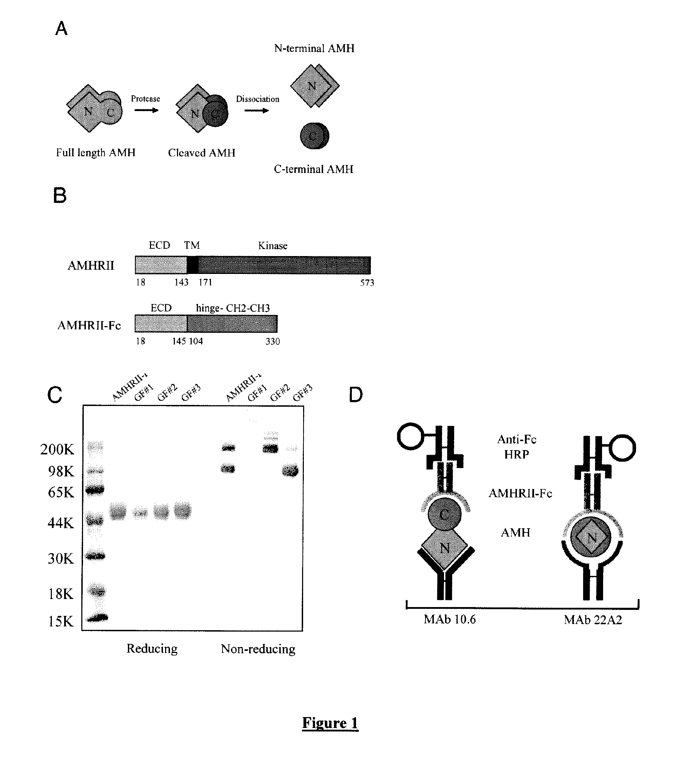

### Figure 1: Generation of an AMHRII-Fc fusion protein and an AMHRII binding ELISA.

(A) Schematic diagram showing processing of AMH.
(B) Schematic diagram showing AMHRII and AMHRII-Fc fusion proteins. The numbering corresponds to amino acid residues in Swiss Prot accession numbers Q16671 (human AMHRII) and P01857 (human Ig gamma-1 chain C region).
(C) SDS-PAGE analysis of the AMHRII-Fc fusion protein and three fractions obtained after gel filtration chromatography. GF#3 contains predominantly dimeric AMHRII-Fc; GF#1-2 contain tetrameric and higher oligomers. Proteins were analyzed under reducing and non-reducing conditions and detected by staining with Coomassie Blue.
(D) Schematic diagram showing the AMHRII binding ELISA with cleaved AMH. Cleaved AMH is captured with either MAb 10.6 against the N-terminus or with MAb 22A2 against the C-terminus. AMHRII-Fc bound to the C-terminus of AMH is detected with a goat anti-human Fc antibody conjugated to HRP.

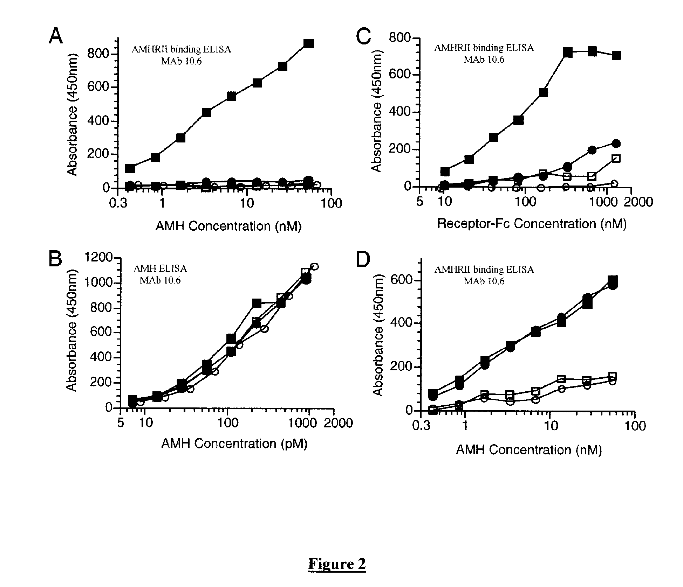

### Figure 2: Selective binding of cleaved AMH to AMHRII-Fc.

(A) AMHRII binding ELISA (MAb 10.6) with cleaved (■), non-cleavable (□), full length (●), or N-terminal AMH (○).
(B) AMH ELISA (MAb 10.6) with cleaved (■), non-cleavable (□), full length (●), or N-terminal AMH (○).
(C) AMHRII binding ELISA (MAb 10.6) with cleaved (■), non-cleavable (□), or full length (●) AMH. TβRII-Fc was substituted for AMHRII-Fc and tested for binding to cleaved AMH (○). AMH concentration: 16 nM.
(D) AMHRII binding ELISA (MAb 10.6): AMHRII-Fc with cleaved (■) or non-cleavable (□) AMH or gel filtration fraction GF#3 with cleaved (●) or non-cleavable AMH (○).

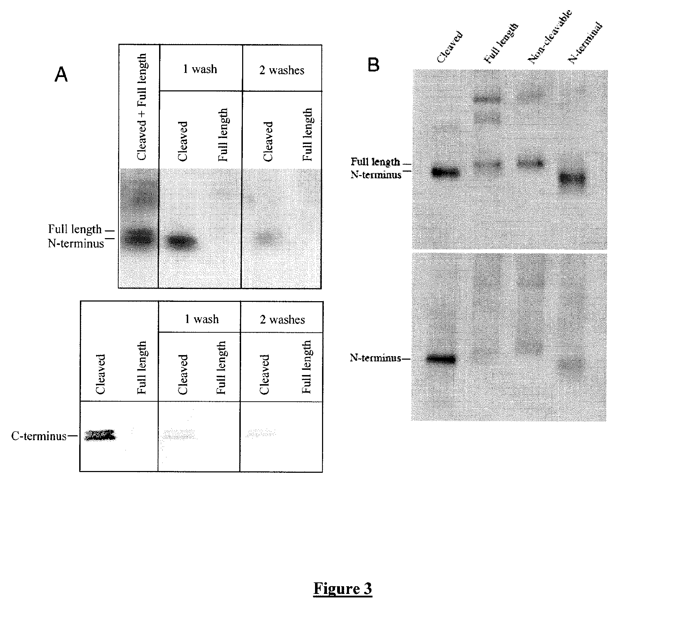

### Figure 3: Binding of AMH to AMHRII-Fc in solution.

(A) Full length and cleaved AMH were precipitated by AMHRII-Fc-Sepharose and washed either once or twice prior to analysis by SDS-PAGE. Top panel: full length and N-terminal AMH detected by western blotting with an anti-AMH polyclonal antibody. Bottom panel: C-terminal AMH detected by protein staining. The lanes on the left in both panels contain samples prior to precipitation.
(B) Cleaved, full length, non-cleavable, and N-terminal AMH were precipitated by AMHRII-Fc-Sepharose, washed once, and analyzed by western blotting with an anti-AMH polyclonal antibody. The top panel shows samples prior to precipitation and the bottom panel shows samples after precipitation.

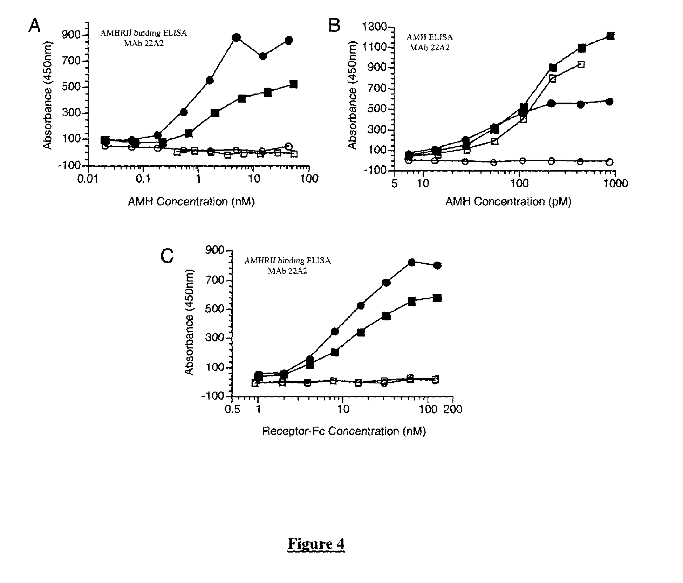

### Figure 4: Binding of C-terminal AMH to AMHRII-Fc.

(A) AMHRII binding ELISA (MAb 22A2) with cleaved (■), non-cleavable (□), or C-terminal (●) AMH, or a C-terminal AMH control (○) (see Experimental Procedures).
(B) AMH ELISA (MAb 22A2) with cleaved (■), non-cleavable (□), or C-terminal (concentration X 50) (●) AMH, or a C-terminal AMH control (○). The anti-human AMH antibody does not recognize C-terminal AMH very well, so 50 fold higher levels of the C-terminal protein were analyzed, relative to cleaved and non-cleavable AMH (i.e. the highest concentration of C-terminal AMH tested was 43 nM, compared to 0.88 nM for cleaved and non-cleavable AMH).
(C) AMHRII binding ELISA (MAb 22A2) with cleaved (■) and C-terminal (●) AMH. TβRII-Fc was substituted for AMHRII-Fc and tested for binding to cleaved (D) and C-terminal AMH (○). AMH concentration: 40 nM.

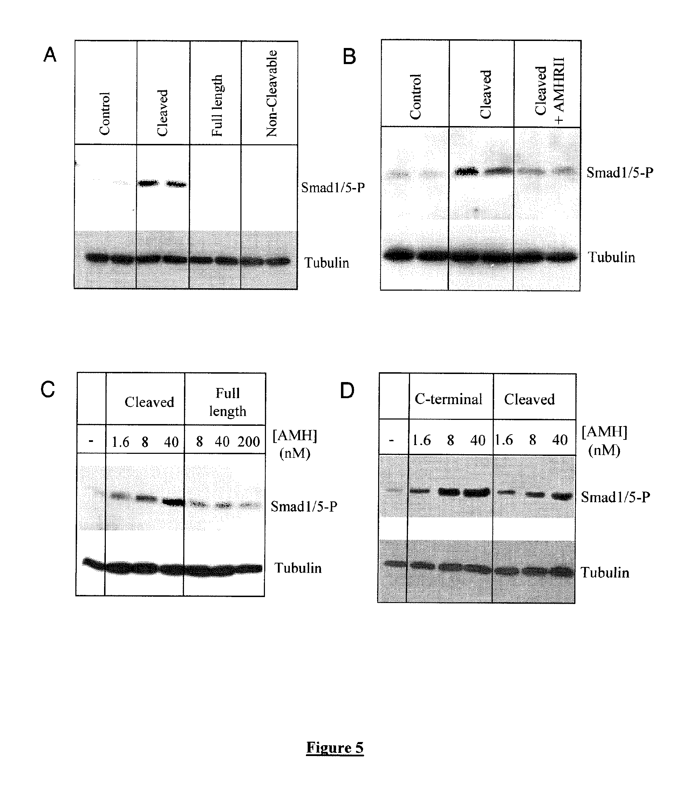

### Figure 5: Full length AMH is inactive and C-terminal AMH is more potent than cleaved AMH in stimulating Smad phosphorylation in SMAT1 cells.

(A) Effects of AMH on Smad phosphorylation. SMAT1 cells were treated for 30 min with the indicated form of AMH (8 nM) and phosphoSmad1/5 and tubulin levels were assessed by western blotting of cell lysates.
(B) Affect of AMHRII-Fc on stimulation of Smad phosphorylation by cleaved AMH. SMAT1 cells were incubated without AMH (control), with cleaved AMH (8 nM), or with cleaved AMH (8 nM) and AMHRII-Fc (1.3 µM).
(C) Dose response comparison between cleaved and full length AMH.
(D) Dose response comparison between cleaved and C-terminal AMH.

### EXAMPLES

### EXPERIMENTAL PROCEDURES

### Generation of the AMHRII-Fc fusion protein.

A cDNA encoding the signal sequence of TβRII (residues 1-23 of Swiss Prot Accession # P37173) fused to the mature extracellular domain of AMHRII (residues 18-145 of Swiss Prot Accession #Q16671) was generated by polymerase chain reaction. It was then ligated to a cDNA encoding the hinge, CH2, and CH3 domains of human IgG1 (residues 104-330 of Swiss Prot Accession #P01857), and cloned into the expression vector pNE001. The plasmid was transfected into 293E cells using effectene (Qiagen) and medium was collected over a two week period at four day intervals. The AMHRII-Fc fusion protein was isolated using Protein A Sepharose. A sample of the fusion protein was further purified by gel filtration using Superdex 200. The human ALK-2, -3, and -6 Fc fusion proteins were obtained from R&D and the human TβRII-Fc protein was the gift of Véronique Bailly (Biogen Idec).

### Preparation of AMH.

Full length, non-cleavable, and N-terminal AMH were purified by immunoaffinity chromatography from culture medium of Chinese Hamster Ovary cells transfected with the wild type human AMH gene or with the R451T or S452stop mutant AMH cDNAs, respectively, and stored in 10 mM HEPES (pH 7.4), 10% glucose, and 300 mM NaCl at - 70°C (Belville et al., 2004). Cleaved AMH was generated by digesting full length AMH with plasmin at a mass ratio of 40:1 for 2 hr at 28°C and the plasmin was removed using a chicken ovoinhibitor affinity column (Gouédard et al., 2000). C-terminal AMH was purified from cleaved AMH on a Sephacryl S-200 column equilibrated with 1 M acetic acid with 0.3 mg/ml PEG 3350 at 4°C (Wilson et al., 1993). Fractions containing the C-terminus were pooled, BSA was added at an equivalent protein concentration, and stored at -70°C. Aliquots of C-terminal AMH were lyophilized and resuspended at a concentration of 100 µg/ml in 1 mM HCl, and PEG 3350 was added to a final concentration of 3 mg/ml. Column buffer (1 M acetic acid and 0.3 mg/ml PEG), lyophilized and resuspended in the same way, was used as a specific negative control for C-terminal AMH. Protein concentrations of AMH preparations were determined by measuring absorbance at 280 nm, using an extinction coefficient of 1.0 ml/mg cm.

### AMHRII binding ELISA.

Dynatech Immulon 2 (96 well) ELISA plates were coated with either mouse anti-AMH MAb 10.6 (which recognizes the N-terminal domain) or MAb 22A2 (which recognizes the C-terminal domain) overnight at 4°C in 50 mM sodium bicarbonate, pH 9.6 (12.5 µg/ml; 50 µl/well). The plates were washed five times with water and then blocked for 2 hr at room temperature using 150 µl/well of block buffer (PBS; 1% BSA; 1% goat serum; used for all dilutions). The block buffer was discarded and AMH was serially diluted down the plate by a factor of two or three. Plates were incubated for 1 hr, followed by five washes with PBS. 50 µl of AMHRII-Fc fusion protein were added to each well at a concentration of 5 µg/ml (64 nM) and incubated for 2 hr. The plates were washed five times with PBS/0.05% Tween-20. Goat anti-human Fc conjugated to HRP (Jackson ImmunoResearch Laboratories) was added at a 1:5000 dilution and the plates were incubated for 50 min. After five washes with PBS/0.05% Tween-20, 50 µl of TMB substrate were added to each well. The reactions were quenched by the addition of 50 µl/well of 2M sulfuric acid. Absorbance at 450 nm was read in a plate reader. In some studies, a fixed amount of AMH (16 or 40 nM; 50 µl/well) was added to the wells, and the AMHRII-Fc fusion protein was serially diluted down the plate.

### AMH ELISA.

The ELISAs to detect AMH were performed similar to the AMHRII binding ELISA, except for the following modifications (Belville et al., 2004). After capturing AMH and washing as above, 50 µl/well of a 1:1000 dilution of rabbit anti-human AMH polyclonal sera was added. One hour later, the plate was washed five times with PBS/0.05% Tween-20 and 50 µl/well of a 1:5000 dilution of goat anti-rabbit IgG antibody conjugated to HRP (Jackson ImmunoResearch Laboratories) was added. After a 50 min incubation, the plate was washed five times with PBS/0.05% Tween-20 and developed as described above.

### Precipitation experiments.

AMHRII-Fc fusion protein (4 mg) was incubated with continuous mixing for 2.5 hr at room temperature with 1 g of cyanogen bromide-activated Sepharose 4B in 2 ml of 200 mM NaCl, 100 mM sodium borate, pH. 8.4. Ethanolamine was added to 100 mM and rocked overnight at 4°C to quench the reaction. The conjugated resin was washed and stored at 4°C in PBS containing 0.02% sodium azide. Prior to precipitation experiments, the resin was washed three times with PBS. 20 µl of the AMHRII-Fc-Sepharose were incubated with 8 µg of AMH in 1 ml of PBS, 100 µg/ml ovalbumin for 60 min at room temperature with agitation. Tween-20 was added to 0.05%, the tube was rocked for 1 min, and the resin was recovered by centrifugation for 1 min. The resin was washed either once or twice with 1 ml of PBS, 100 µg/ml ovalbumin, 0.05% Tween-20 for 1 min and recovered by centrifugation for 1 min. 20 µl of 2X non-reducing sample buffer was added, the tube was heated at 65°C for 10 min, and aliquots of the recovered supernatant were subjected to SDS-PAGE (4-20% gradient gel). C-terminal AMH was detected using an imidazole-SDS-Zn reverse staining method (Ortiz et al., 1992). Full length, non-cleavable, and N-terminal AMH were detected by western blotting using rabbit anti-human AMH polyclonal sera (1:1000 dilution) and goat anti-rabbit IgG antibody conjugated to HRP (Jackson ImmunoResearch Laboratories; 1:5000 dilution). Proteins were visualized with the ECL Plus Kit Detection System (Amersham Pharmacia Biotech, Piscataway, NJ).

### SMAT1 cell experiments.

Smad phosphorylation was determined as previously described (Gouédard et al., 2000). Briefly, SMAT1 cells were seeded into 6 well tissue culture plates at approximately 50% density. The next day the cells were washed, medium without serum was added for 1 hr, and then AMH was added in culture medium without serum for 30 min. The cells were washed and solubilized in 200 µl of lysis buffer (20 mM Tris-HCl, pH 7.5, 150 mM NaCl, 1% (V/V) Triton X-100) containing 1 mM phenylmethylsulfonyl fluoride, a proteinase inhibitor mixture (Sigma-Aldrich, St Louis, MO) and a phosphatase inhibitor cocktail (Calbiochem Merck Biosciences, Darmstadt, Germany). The lysates were cleared by centrifugation and supernatants were analyzed by SDS-PAGE followed by western blotting with a rabbit anti-phosphoSmad1, 5 MAb (Cell Signaling; 1:1000 dilution) and a goat anti-rabbit IgG antibody conjugated to HRP (Jackson ImmunoResearch Laboratories; 1:5000 dilution). The membranes were stripped and reprobed with a mouse anti-tubulin MAb (clone B-5-1-2, Sigma-Aldrich, St. Louis, MO).

### RESULTS

### Soluble AMHRII binds to cleaved AMH but not to full length AMH.

A soluble form of the type II AMH receptor, AMHRII, was produced as shown in Figure 1B. The fusion protein containing the extracellular domain of human AMHRII and the Fc domain of human IgG1 was expressed in 293E cells and purified using Protein A Sepharose. The gel analysis shown in Figure 1C indicated that the fusion protein consists mostly of dimer, but that a significant domain of the purified protein appears to be tetrameric and higher order multimers. In order to rule out problems that might occur with higher oligomeric forms of the fusion protein, the purified AMHRII-Fc was subjected to gel filtration chromatography allowing the isolation of an almost pure dimeric fusion protein (GF#3 in Figure 1C). The other two fractions contain tetramers and higher order multimers.

The binding of AMH to AMHRII-Fc was assessed using the ELISA format shown in Figure 1D. Plates were coated with the mouse monoclonal antibody MAb 10.6, which recognizes an epitope on the N-terminal domain of human AMH. Four different forms of AMH were then captured by the monoclonal antibody: full length AMH, cleaved AMH, N-terminal AMH (Figure 1A), and non-cleavable AMH (Belville et al., 2004; Wilson et al., 1993). Full length AMH contains about 90% 140 kDa homodimer and 10% non-covalent complex of the N- and C-terminal homodimers, while cleaved AMH has been digested with plasmin and contains 100% non-covalent complex. N-terminal AMH contains only the N-terminal homodimer. Non-cleavable AMH contains only 140 kDa homodimer with R451T mutations at the monobasic cleavage sites, rendering it resistant to cleavage with plasmin and biologically inactive. After incubation of each of these captured proteins with the AMHRII-Fc fusion protein, bound AMHRII-Fc was detected with an anti-human Fc secondary antibody. As shown in Figure 2A, only cleaved AMH showed significant binding to the soluble AMHRII protein. To demonstrate that all four proteins were present in the assay at equivalent levels, parallel plates that were coated with the MAb 10.6 and treated with the four AMH proteins, were detected with an anti-AMH rabbit polyclonal antibody and an anti-rabbit Fc secondary antibody. Figure 2B shows that almost identical amounts of all four proteins were captured by the anti-N-terminal AMH antibody. Thus the lack of binding of AMHRII-Fc to full length, non-cleavable, and N-terminal AMH is not due to a failure of these proteins to be captured by the anti-AMH monoclonal antibody.

The assays shown in Figure 2A were performed with dilutions of AMH and a fixed concentration of AMHRII-Fc. To investigate whether the interaction of AMHRII-Fc with cleaved AMH was saturable, the format was switched so that a fixed concentration of AMH was captured by the MAb 10.6 antibody, and the AMHRII-Fc concentration was varied. Again, as shown in Figure 2C, only cleaved AMH showed significant binding to AMHRII-Fc and this binding was saturable. The small amount of binding of full length AMH seen in this assay at high AMHRII-Fc concentrations may be due to the small amount (approximately 10%) of cleaved AMH in the full length AMH preparation. Another control included in this experiment shows that a TGF-β type II receptor (TβRII)-Fc fusion protein did not bind to cleaved AMH, consistent with AMH having no effect on TGF-β responsive cells and confirming that the interaction between AMHRII-Fc and AMH is specific. A final control shown in Figure 2D indicates that the AMHRII-Fc preparation containing some tetrameric protein behaved identical to gel filtration fraction GF#3, which is almost completely dimeric (Figure 1C). Thus the binding of AMHRII-Fc to cleaved AMH is not the result of higher oligomeric molecules within the AMHRII-Fc preparation.

### Soluble AMHRII binds to cleaved AMH in solution.

In order to test whether the interaction between AMHRII-Fc and cleaved AMH can also be observed in solution, precipitation experiments were performed and the results are shown in Figure 3. AMHRII-Fc coupled to Sepharose was able to precipitate cleaved AMH, but not full length AMH. As shown in Figure 3A, both the N-terminus (detected by western blot in the top panel) and the C-terminus (detected by staining in the lower panel) were precipitated by the AMHRII-Fc. In contrast, no full length protein was detectable by western blotting (Figure 3A; top panel). Extra washing decreased the amount of precipitated N-terminus (Figure 3A; top panel) but did not affect the level of precipitated C-terminus (Figure 3A; bottom panel), suggesting that the C-terminus does not require the N-terminus for binding to AMHRII-Fc. In Figure 3B, the N-terminal and non-cleavable AMH proteins were tested and only minimal amounts of these proteins and full length AMH were precipitated by the AMHRII-Fc Sepharose, compared to cleaved AMH.

### Soluble AMHRII binds to isolated C-terminal AMH.

To investigate whether soluble AMHRII could bind to the isolated C-terminal homodimer of AMH, the AMHRII binding ELISA format was changed and an antibody that recognizes the C-terminal domain (MAb 22A2) was used to capture AMH (Figure 1D). As shown in Figure 4A, binding of AMHRII was observed to both C-terminal and cleaved AMH, while no binding of AMHRII was observed to non-cleavable AMH or to a negative control for C-terminal AMH (see Experimental Procedures). To demonstrate that the AMH proteins were present in the assay at equivalent levels, they were directly detected with an anti-AMH rabbit polyclonal antibody and an anti-rabbit Fc secondary antibody. Figure 4B shows that almost identical amounts of cleaved and non-cleavable AMH were captured by the anti-C-terminal AMH antibody. However, a lower signal was observed for C-terminal AMH because the anti-AMH polyclonal antibody shows more reactivity toward the N-terminal domain that the C-terminal domain. Although this makes it difficult to directly compare the binding profiles of AMHRII to cleaved and C-terminal AMH in Figure 4A, the protein concentrations of both AMH preparations have been firmly established and equal amounts (on a molar basis) were introduced into the assay. Thus it is reasonable to assume that equivalent amounts of the two proteins were captured.

To confirm these results the format was switched and a fixed concentration of AMH was captured by the MAb 22A2 antibody, while the AMHRII-Fc concentration was varied. As shown in Figure 4C, AMHRII-Fc bound to both cleaved and C-terminal AMH, but no binding of TβRII-Fc was observed to either of these proteins. Again a small difference was observed between the binding of AMHRII-Fc to cleaved and C-terminal AMH. C-terminal AMH bound more AMHRII-Fc than cleaved AMH, but the concentrations of AMHRII-Fc at which half-maximal binding to the two proteins occurred were similar.

### Cleaved and C-terminal AMH stimulate phosphorylation of Smads.

A mouse Sertoli cell line, SMAT1 (Dutertre et al., 1997), was used to assess which forms of AMH can stimulate an intracellular signaling pathway. The SMAT1 cell line expresses AMHRII and all three type I receptors, and has been shown to be responsive to AMH as evident by phosphorylation of Smad1, 5, or 8, the first components in the signal transduction pathway (Belville et al., 2005). Figure 5A shows the results of a comparison of cleaved, full length, and non-cleavable AMH, all present at a concentration of 8 nM. After a 30 minute incubation, only cleaved AMH induced phosphorylation of Smads. Figure 5B shows that the soluble AMHRII fusion protein partially blocked Smad phosphorylation induced by cleaved AMH, indicating that Smad phosphorylation is dependent on an interaction between AMH and AMHRII on the surface of the SMAT1 cells.

Figures 5C and D show a dose response comparison between cleaved AMH and either full length AMH or C-terminal AMH, respectively. The results of panel C confirm the results of panel A: only cleaved AMH had a significant effect on inducing phosphorylation of Smads. However, the results of panel D show that C-terminal AMH was more potent than cleaved AMH at stimulating phosphorylation of Smads: a lower concentration of the C-terminal AMH (8 nM) produced a higher level of phosphorylated Smads than a higher concentration of cleaved AMH (40 nM). Thus, C-terminal AMH is more potent than cleaved AMH in stimulating an intracellular pathway, indicating that release of the N-terminal domain from the C-terminal domain may be required for maximal activity.

In conclusion, we have used a soluble type II receptor and an AMH responsive cell line to identify the form of AMH that can bind to its type II receptor and rapidly stimulate phosphorylation of Smads 1 and 5, the first step in the intracellular pathway. Using an ELISA format, we were able to directly detect an interaction between AMH and AMHRII. While cleaved and C-terminal AMH could bind to AMHRII and stimulate phosphorylation of Smads, full-length AMH could not, indicating that AMH must be cleaved for it to interact with its type II receptor, and that dissociation of the pro region is not required for this interaction.

### REFERENCES

All the cited references are incorporated by reference.

Al-Qahtani A, Muttukrishna S, Appasamy M, Johns J, Cranfield M, Visser JA, Themmen AP, Groome NP. (2005). Development of a sensitive enzyme immunoassay for anti-Müllerian hormone and the evaluation of potential clinical applications in males and females. Clin Endocrinol 2005 Sep;63(3):267-73.

Behringer, R.R., Finegold, M.J., and Cate, R.L. (1994). Mullerian-inhibiting substance function during mammalian sexual development. Cell 79, 415-425.

Belville, C., Van Vlijmen, H., Ehrenfels, C., Pepinsky, B., Rezaie, A.R., Picard, J.Y., Josso, N., di Clemente, N., and Cate, R.L. (2004). Mutations of the anti-mullerian hormone gene in patients with persistent mullerian duct syndrome: biosynthesis, secretion, and processing of the abnormal proteins and analysis using a three-dimensional model. Mol Endocrinol 18, 708-721.

Blanchard, M.G., and Josso, N. (1974). Source of the anti-Mullerian hormone synthesized by the fetal testis: Mullerian-inhibiting activity of fetal bovine Sertoli cells in tissue culture. Pediatr Res 8, 968-971.

Chang, H., Brown, C.W., and Matzuk, M.M. (2002). Genetic analysis of the mammalian transforming growth factor-beta superfamily. Endocr Rev 23, 787-823.

Clarke, T.R., Hoshiya, Y., Yi, S.E., Liu, X., Lyons, K.M., and Donahoe, P.K. (2001). Mullerian inhibiting substance signaling uses a bone morphogenetic protein (BMP)-like pathway mediated by ALK2 and induces SMAD6 expression. Mol Endocrinol 15, 946-959.

di Clemente, N., Ghaffari, S., Pepinsky, R.B., Pieau, C., Josso, N., Cate, R.L., and Vigier, B. (1992). A quantitative and interspecific test for biological activity of anti-mullerian hormone: the fetal ovary aromatase assay. Development 114, 721-727.

Dutertre, M., Rey, R., Porteu, A., Josso, N., and Picard, J.Y. (1997). A mouse Sertoli cell line expressing anti-Mullerian hormone and its type II receptor. Mol Cell Endocrinol 136, 57-65.

Gentry, L.E., and Nash, B.W. (1990). The pro domain of pre-pro-transforming growth factor beta 1 when independently expressed is a functional binding protein for the mature growth factor. Biochemistry 29, 6851-6857.

Gouédard, L., Chen, Y.G., Thevenet, L., Racine, C., Borie, S., Lamarre, I., Josso, N., Massague, J., and di Clemente, N. (2000). Engagement of bone morphogenetic protein type IB receptor and Smad1 signaling by anti-Mullerian hormone and its type II receptor. J Biol Chem 275, 27973-27978.

Gustafson,M.L., Lee,M.M., Scully,R.E., Moncure,A.C., Hirakawa,T., Goodman,A., Muntz,H.G., Donahoe,P.K., MacLaughlin,D.T., and Fuller,A.F. (1992). Müllerian inhibiting substance as a marker for ovarian sex-cord tumor. New Engl J Med 326, 466-471.

Hoshiya Y, Gupta V, Segev DL, Hoshiya M, Carey JL, Sasur LM, Tran TT, Ha TU, Maheswaran S. (2003) Mullerian Inhibiting Substance induces NFkB signaling in breast and prostate cancer cells. Mol Cell Endocrinol 211(1-2), 43-49.

Imbeaud, S., Faure, E., Lamarre, I., Mattei, M.G., di Clemente, N., Tizard, R., Carre-Eusebe, D., Belville, C., Tragethon, L., Tonkin, C., et al. (1995). Insensitivity to anti-mullerian hormone due to a mutation in the human anti-mullerian hormone receptor. Nat Genet 11, 382-388.

Jamin, S.P., Arango, N.A., Mishina, Y., Hanks, M.C., and Behringer, R.R. (2002). Requirement of Bmpr1a for Mullerian duct regression during male sexual development. Nat Genet 32, 408-410.

Josso, N., and di Clemente, N. (2003). Transduction pathway of anti-Mullerian hormone, a sex-specific member of the TGF-beta family. Trends Endocrinol Metab 14, 91-97.

Josso, N., Picard, J.Y., Rey, R., and di Clemente, N. (2006). Testicular anti-Mullerian hormone: history, genetics, regulation and clinical applications. Pediatr Endocrinol Rev 3, 347-358.

La Marca A. and Volpe A. 2007. The anti mullerian hormone and ovarian cancer. Human Reproduction Update 13(3):265-73.

Long,W.Q., Ranchin,V., Pautier,P., Belville,C., Denizot,P., Cailla,H., Lhommé,C., Picard,J.Y., Bidart,J.M., and Rey,R. (2000). Detection of minimal levels of serum anti-Müllerian hormone during follow- up of patients with ovarian granulosa cell tumor by means of a highly sensitive ELISA. J Clin Endocrinol Metab 85, 540-544.

Mishina, Y., Rey, R., Finegold, M.J., Matzuk, M.M., Josso, N., Cate, R.L., and Behringer, R.R. (1996). Genetic analysis of the Mullerian-inhibiting substance signal transduction pathway in mammalian sexual differentiation. Genes Dev 10, 2577-2587.

Ortiz, M.L., Calero, M., Fernandez Patron, C., Patron, C.F., Castellanos, L., and Mendez, E. (1992). Imidazole-SDS-Zn reverse staining of proteins in gels containing or not SDS and microsequence of individual unmodified electroblotted proteins. FEBS Lett 296, 300-304.

Orvis, G.D., Jamin, S.P., Kwan, K.M., Mishina, Y., Kaartinen, V.M., Huang, S., Roberts, A.B., Umans, L., Huylebroeck, D., Zwijsen, A., et al. (2008). Functional Redundancy of TGF-beta Family Type I Receptors and Receptor-Smads in Mediating Anti-Mullerian Hormone-Induced Mullerian Duct Regression in the Mouse. Biol Reprod.

Pieretti-Vanmarcke R., Donahoe P.K., Szotek P., Manganaro T., Lorenzen M.K., Lorenzen J., Conolly D.C., Halpern E.F. and MacLaughlin D.T. (2006a). Recombinant human mullerain inhibiting substance inhibits long-term growth of MIS type II receptor-directed transgenic mouse ovarian cancers in vivo. Clin. Cancer Res. 12(5):1593-1598.

Pieretti-Vanmarcke R., Donahoe P.K., Pearsall A.L., Dinulescu D.M., Conolly D.C., Halpern E.F., Seiden M.V., and MacLaughlin D.T. (2006b). Mullerian inhibiting substance enhances subclinical doses of chemotherapeutic agents to inhibit human and mouse ovarian cancer. PNAS 103, 46: 17426-17431.

Pepinsky, R.B., Sinclair, L.K., Chow, E.P., Mattaliano, R.J., Manganaro, T.F., Donahoe, P.K., and Cate, R.L. (1988). Proteolytic processing of mullerian inhibiting substance produces a transforming growth factor-beta-like fragment. J Biol Chem 263, 18961-18964. Renaud EJ, MacLaughlin DT, Oliva E, Rueda BR, Donahoe PK. (2005). Endometrial cancer is a receptor-mediated target for Mullerian Inhibiting Substance. Proc Natl Acad Sci U S A Jan 4;102(1):111-6.

Shi, Y., and Massagué, J. (2003). Mechanisms of TGF-beta signaling from cell membrane to the nucleus. Cell 113, 685-700.

Tüttelmann,F., Dykstra,N., Themmen,A.P.N., Visser,J.A., Nieschlag,E., and Simoni,M. (2008). Anti-Müllerian hormone in men with normal and reduced sperm concentration and men with maldescended testes. Fert Steril In Press, Corrected Proof.

Visser, J.A., Olaso, R., Verhoef-Post, M., Kramer, P., Themmen, A.P., and Ingraham, H.A. (2001). The serine/threonine transmembrane receptor ALK2 mediates Mullerian inhibiting substance signaling. Mol Endocrinol 15, 936-945.

Wilson, C.A., di Clemente, N., Ehrenfels, C., Pepinsky, R.B., Josso, N., Vigier, B., and Cate, R.L. (1993). Mullerian inhibiting substance requires its N-terminal domain for maintenance of biological activity, a novel finding within the transforming growth factor-beta superfamily. Mol Endocrinol 7, 247-257.

## Claims

1. A method for detecting or quantifying the presence of at least one biologically active form of Anti-Müllerian Hormone (AMH) in a sample, said method comprising detecting the presence of cleaved AMH or C-terminal AMH in said sample.

2. The method according to claim **1,** said method comprising detecting at least one biologically active form of Anti-Müllerian Hormone (AMH) with a binding partner comprising the extra-cellular domain of type II AMH receptor (AMHRII).

3. The method according to claim **1** or **2,** wherein said sample is a whole blood sample, a serum sample, a plasma sample, a follicular fluid sample or a seminal fluid sample.

4. The method according to anyone of claims **1** to **3,** wherein the binding partner is the extra-cellular domain of AMRII.

5. The method according to anyone of claims **1** to **4,** wherein the binding partner is a protein fusion comprising the extra-cellular domain of AMRII.

6. The method according to anyone of claims **1** to **5,** comprising detecting at least one biologically active form of AMH in a sample obtained from said subject, with a binding partner comprising the extra-cellular domain of AMHRII and an anti-AMH antibody that does not prevent the interaction between the biologically active forms of AMH and the extra-cellular domain of AMHRII.

7. The method according to claim **6**, wherein the detection of at least one biologically active form of AMH is achieved by a non-competitive immunoassay.

8. The method according to claims **6** or **7**, wherein the anti-AMH antibody is an antibody directed to an epitope present in the N-terminal domain of AMH.

9. The method according to anyone of claims **6** to **8,** wherein the anti-AMH antibody is an antibody directed to an epitope present in the C-terminal domain of AMH.

10. A method for determining the fertility of a subject, said method comprising quantifying at least one biologically active form of AMH in a sample obtained from said subject according to anyone of claims **1** to **9.**

11. A method for determining and/or monitoring the presence of a cancer in a subject in need thereof, said method comprising quantifying at least one biologically active form of AMH in a sample obtained from said subject according to anyone of claims **1** to **9.**

12. The method according to claim **11,** wherein said method is for diagnosing and/or monitoring a cancer, said cancer being a neoplasm stemming from granulosa cell tumours, an ovarian cancer, an uterine cancer, a breast cancer, a prostate cancer.

13. A kit for use in the method according to anyone of claims **1** to **12,** comprising as separate components:
- a binding partner comprising the extra-cellular domain of AMHRII, and
- an anti-AMH antibody that does not prevent the interaction between the biologically active forms of AMH and the extra-cellular domain of AMHRII.

14. The kit according to claim **15,** wherein the binding partner or the anti-AMH antibody is coated to a solid support and/or the binding partner or the anti-AMH antibody is labelled.

15. Cleaved AMH and/or C-terminal AMH for treating cancer in a subject in need thereof.
